# EUROPEAN PATENT APPLICATION

(11) **EP 1 348 430 A1**
(43) Date of publication of application: **01.10.2003**
(21) Application number: 03004628.8
(22) Date of filing: 03.03.2003
(51) Int. Cl.: A61K 31/335, A61K 9/16, A61K 9/50, A61K 9/51, A61K 47/42

(54) **Improved paclitaxel-based antitumor formulation**

(30) Priority: 29.03.2002 IT MI20020680
(71) Applicant: ACS DOBFAR S.p.A., 20067 Tribiano (Milano) (IT)
(72) Inventor: Zenoni, Maurizio, 20067 Paullo (MI) (IT); Maschio, Simone, 00199 Rome (IT)
(74) Representative: Frignoli, Luigi

(57) **Abstract**

Antitumor formulation based on nanoparticles of paclitaxel and human serum albumin as obtained by the addition of a biocompatible acid to an aqueous albumin solution before this is mixed with paclitaxel during the nanoparticle production process, the injectable solutions of this formulation having a pH between 5.4 and 5.8 and having stability and inalterability with time.

## Description

The present invention relates to an antitumor formulation based on paclitaxel and albumin nanoparticles able to give injectable reconstituted aqueous mixtures having high inalterability with time.

Paclitaxel is a natural substance well known in literature, with important antitumor activity. Its poor water solubility makes it difficult to administer to man, for which reason various systems have been developed to render it injectable.

Bristol Myers Squibb (BMS) have conceived and patented a composition, known by the name of TAXOL®, in which the paclitaxel is emulsified with cremophor which induces various side effects in the patient (Lorenz et al., Agents Action 7, 63-67 (1987); Weiss et al., J. Clin. Oncol. 8, 1263 (1990)). The BMS formulation also involves lengthy administration times due to the dilution of the active principle.

To obviate the described drawbacks, BMS have patented (EP-A-0584001, EP-A-0783885, EP-A-0783886, US 5641803, US 5670537) formulations of TAXOL® with the same dose of paclitaxel but with other excipients able to prevent strong anaphylactic reactions. However in all cases patient administration must be effected very slowly, over a period of about 3 hours.

To prevent the side effects of TAXOL®, the cremophor was replaced with human serum albumin (HSA) in view of its biocompatibility and its considerable capacity to bind to the paclitaxel (Kumar et al., Res. Comm. in Chem. Path. and Pharm., 80 (3), 337-343 (1993); Paal et al., Eur. J. Biochem. 268, 2187-2191 (2001)). The property of HSA to form microspheres containing active principles dissolved in organic solvents insoluble in water (Kramer et al., J. Pharm. Sci. 63, 1646-1647 (1974); Grinstaff and Suslick, J. Am. Chem. Soc. 112, 7807-7809 (1990); Grinstaff and Suslick, Polym. Prepr. 32, 255-256 (1991)) has also enabled the development of systems for administering paclitaxel in higher concentrations than with TAXOL®.

Injectable nanoemulsions of paclitaxel and HSA can be obtained by known ultrasonication, high pressure homogenization and microfluidization techniques (Alleman et al., Eur. J. Pharm. Biopharm. 39 (5), 173-191 (1993)).

On the basis of these elements and by using the aforestated ultrasonication and high pressure homogenization techniques, the American company VivoRx Pharmaceuticals Inc. has developed the formulation CAPXOL® containing paclitaxel and HSA.

In US 5439686, US 5498421, US 5560933 and the corresponding WO 94/18954, VivoRx claims microparticles of paclitaxel and HSA prepared using ultrasonication techniques, to give particles of mean size (MPS) < 10 microns. The preparation methods described in these patents cannot be used on an industrial scale, and moreover the microparticles thus obtained have too high an MPS, which makes them unsuitable and unusable for administration to patients.

This was well known to the said VivoRx, which then in US 5916596 and US 6096331 and in WO 98/14174 and WO 99/00113 described and claimed sterile nanoemulsions of paclitaxel and HSA obtained by reconstituting with sterile aqueous 0.9% NaCl solution lyophilized powders with MPS < 0.2 microns. These nanoemulsions, which are obtained using high pressure homogenization, as described in the cited patents, are stated to have high stability, where the term "stability" means that the MPS is constant with time and that nanoparticle precipitation is absent (US 6096331, Ex. 11).

Using maximum care, the present applicants have several times reproduced the examples of the aforestated patents, in particular Examples 1, 5 and 6 of US 5916596, without ever obtaining the result specified in the examples and claimed in the patent. Having prepared the mixtures as described, then processing them with an Avestin homogenizer within the pressure range recommended in US 5916596, nanoemulsions at pH=6.7 were obtained which, when evaporated in a rotavapor as reported in the said patent, always provided nanoemulsions with MPS of about 0.2 microns (increase of MPS > 0.02 microns after evaporation) which are poorly stable in their formulations in injectable physiological solutions (increase in MPS of about 0.05 microns and tendency to sediment in about 12 hours) and difficult to filter through 0.22 microns filters for their sterilization, in contrast to that stated in the said patent.

The present applicants have made the most careful attempts to effect filtration with the membranes described in US 5916596, but these attempts have always failed, with clogging of the filters and paclitaxel yields always < 30%, in contrast to the 70-100% declared. Moreover the stability (evaluated in accordance with the teachings of Example 11 of US 6096331) of the products prepared by the method just described, then lyophilized and reconstituted as reported in US 5916596 and US 6096331 has never reached 24 hours (hence much less than the 72 hours declared in the patents).

The main object of the present invention is therefore to provide an antitumor formulation consisting of nanoparticles of paclitaxel and human serum albumin, which with a physiological solution enables injectable reconstituted mixtures to be formed in which said particles have a stability (in the aforestated sense) considerably greater than that possible in the known art, and specifically a stability exceeding 24 hours.

This and further objects are attained by a formulation consisting of a lyophilized powder of nanoparticles of paclitaxel and human serum albumin, in which the paclitaxel is present in a quantitity between 1% and 20% and the albumin between 60% and 98%, the percentages being by weight and the mean nanoparticle size being less than 0.2 microns, characterized in that said lyophilized powder contains between 1% and 20% by weight of biocompatible salts obtained by salification of at least one biocompatible acid or due to the presence of at least one biocompatible acid buffer substance, the acid or the buffer substance being present in a quantity such that the pH of a reconstituted aqueous injectable mixture of the powder is between 5.4 and 5.8.

The presence of the salts is due to the fact that an acid buffer substance is (as chemists well know) formed by an acid and a salt thereof and that some basic groups present in albumin are salified by the acids, therefore providing a mixture having a pH lower than a typical pH of albumin, i.e. 6.79-6.89 according to Merck Index, 13^{th} Ed. page 1519.

Experiments have shown that if use is made of an acid buffer substance (such as a mixture of citric acid and sodium citrate), the results are not so good as with the use of the acid alone (citric acid or other biocompatible acid), as far as the abovementioned stability is concerned.

Obviously, the pH of the lyophilized powder can be easily measured after water has been added to form an aqueous mixture with it. The acidic nanoparticles have been studied showing that also water is present therein: the amount of water in the powder is up to 5% (w/w), usually about 2% to 4.5% (w/w). As a consequence, even the above mentioned nanoparticles containing water form part of the present invention.

The invention also relates to injectable reconstituted aqueous mixtures of such formulations, in which the paclitaxel is present at a concentration between 0.1 and 3 mg/ml, preferably between 0.5 and 2.5 mg/ml.

The formulations of the invention may be obtained by mixing a sterile aqueous solution of human serum albumin (HSA) with a sterile solution of paclitaxel and treating this mixture in accordance with the teachings of the aforesaid Vivorx patents, but differing from such teachings in the fact that to the aqueous HSA solution, before it is mixed with paclitaxel, at least one biocompatible acid or acid buffer substance is added in a quantity sufficient to bring the pH of the solution to between 5.4 and 5.8, preferably between 5.5 and 5.7.

The biocompatible acids may be chosen from the group consisting of HCl, citric acid, phosphoric acid, acetic acid, biocompatible organic and inorganic acids.

The same formulations may be obtained also by a process according to which an aqueous mixture containing paclitaxel and albumin at a temperature between 0°C and 40°C is subjected to homogenization treatment at high pressure between 9000 and 40000 psi, to give a nanoemulsion which is frozen between-20°C and -80°C and is finally lyophilized by heating at a temperature between +20°C and +35°C, wherein said aqueous mixture is obtained under sterile conditions by dissolving said albumin in sterile water to a concentration between 2% and 3% (w/v), then adding to said albumin solution between 2% and 4% (v/v) of chloroform and then paclitaxel in sterile powder form in a quantity between 5.40% and 15.0%, preferably between 5.60% and 13.7%, by weight on the weight of the albumin present in the solution, at least one biocompatible acid or acid buffer substance being added to said albumin solution before adding the paclitaxel in a quantity sufficient to bring the pH of the mixture to between 5.4 and 5.8, preferably between 5.5 and 5.7.

It may be noted that the use of paclitaxel in sterile powder form in the latter process not only greatly simplifies the plant itself and the process compared with the known art and enables the time required to complete the mixing of the various components before the homogenization treatment to be considerably shortened, but also enables better final yields to be obtained and simplifies the conditions to be observed in order to obtain the desired sterile lyophilized powders.

The results obtained with the use of the formulations according to the present invention are totally unexpected and surprising, because they are in contrast to the teachings of the art which provides for the use of HSA solutions of pH values resulting from the dilution of injectable solutions of said albumin complying with FDA specifications, hence at pH=6.9±0.5 (see Examples 1, 5 and 6 of US 5916596). In contrast to the teachings of the known art, it has been discovered that at pH values between 5.4 and 5.8 a stability of greater than 24 hours can be obtained for the reconstituted lyophilized products.

To clarify the understanding of the characteristics of the present invention, some non-limiting examples of its implementation will now be described.

### EXAMPLE 1

### Preparation of a formulation with HCl and paclitaxel dissolved in cloroform

An injectable aqueous 25% (w/v) HSA solution in accordance with FDA specifications (pH=6.9±0.5) is diluted to 3% (w/v) with sterile demineralized water, the pH being corrected to 5.6 with 1M HCl which salifies some basic groups present in albumin. 40 ml of said solution, previously sterilized, are mixed with 1.2 ml of a sterile solution of paclitaxel (59.0 mg/ml) in CHCl₃, after which the mixture is processed in a homogenizer (suitably sterilized) at high pressure (9000-40000 psi) until a nanoemulsion (MPS < 0.2 microns) is obtained, this being frozen to -25°C and lyophilized for 60 hours under sterile conditions, while raising the temperature to +20°C.

The powder obtained, containing 4.25 % (w/w) of paclitaxel and 3.6 (w/w) of water, is reconstituted with an aqueous 0.9% NaCl solution to a paclitaxel concentration of 2 mg/ml. The formulation obtained has an MPS of 0.16 microns, pH=5.6, and a stability > 24 hours.

Equivalent results were obtained by using phosphoric acid instead of HCl.

### EXAMPLE 2

### Preparation of a formulation with citric acid and paclitaxel dissolved in cloroform

An injectable aqueous 25% (w/v) HSA solution in accordance with FDA specifications (pH=6.9±0.5) is diluted to 2.5% (w/v) with sterile demineralized water, the pH being corrected to 5.5 with sterile citric acid which salifies some basic groups present in albumin. 60 ml of said solution are mixed with 1.7 ml of a sterile solution of 60.0 mg/ml of paclitaxel in CHCl₃, after which the mixture is processed in a homogenizer (suitably sterilized) at high pressure (9000-40000 psi) until a nanoemulsion (MPS < 0.2 microns) is obtained, this being rapidly frozen to -40°C and lyophilized for 55 hours under sterile conditions, while raising the temperature to +35°C.

The powder obtained, containing 5.2% of paclitaxel and 4.9% (w/w) of water, is reconstituted with an aqueous 0.9% NaCl solution to a paclitaxel concentration of 2 mg/ml. The formulation obtained has an MPS of 0.17 microns, pH=5.5, and a stability > 24 hours.

### EXAMPLE 3

### Preparation of a formulation with HCl and paclitaxel dissolved in cloroform

An injectable aqueous 25% HSA solution in accordance with FDA specifications is diluted to 3% (w/v) with sterile demineralized water, the pH being corrected to 5.6 with 1M HCl which salifies some basic groups present in albumin. 60 ml of said solution, suitably sterilized, are mixed with 1.5 ml of a sterile solution of 75 mg/ml of paclitaxel in CHCl₃, after which the mixture is processed in a homogenizer (suitably sterilized) at high pressure (9000-40000 psi) until a nanoemulsion (MPS < 0.2 microns) is obtained, this being frozen to -50°C and lyophilized for 50 hours under sterile conditions, while raising the temperature to +30°C.

The powder obtained, containing 4.41% of paclitaxel and 3.8% (w/w) of water, is reconstituted with an aqueous 0.9% NaCl solution to a paclitaxel concentration of 2.5 mg/ml. The formulation obtained has an MPS of 0.175 microns, pH=5.6, and a stability > 24 hours.

By repeating the same procedure but without adding HCl and hence working at about pH 6.5, a formulation is obtained with an MPS of 0.24 microns and a stability of about 10 hours.

### EXAMPLE 4

### Preparation of a formulation with citric acid from a paclitaxel solution

An injectable aqueous 25% (w/v) HSA solution in accordance with FDA specifications is diluted to 3% (w/v) with sterile demineralized water, the pH being corrected to 5.4 with sterile citric acid which salifies some basic groups present in albumin.

50 ml of said solution are mixed under vigorous agitation for at least 40 minutes with 1.25 ml of a sterile solution of paclitaxel in chloroform (75 mg/ml).

The mixture is processed in a homogenizer (suitably sterilized) at high pressure (9000-40000 psi) until a nanoemulsion (MPS < 0.2 microns) is obtained, this being rapidly frozen to -30°C and lyophilized for 57 hours under sterile conditions, while raising the temperature to +35°C.

The powder obtained, containing 5.00% (w/w) of paclitaxel and 4.3 (w/w) of water, is reconstituted with an aqueous 0.9% NaCl solution to a paclitaxel concentration of 2 mg/ml. The formulation obtained has an MPS of 0.19 microns, pH=5.4, and a stability > 24 hours.

Equivalent results are obtained by using acetic acid instead of citric acid.

### EXAMPLE 5

### Preparation of a formulation with HCl and paclitaxel in powder form

An injectable aqueous 25% (w/v) HSA solution in accordance with FDA specifications (pH=6.9±0.5) is diluted to 3% (w/v) with sterile demineralized water, the pH being corrected to a value of 5.6 with 1M HCl which salifies some basic groups present in albumin.

57 ml of said solution, previously sterilized, are mixed under vigorous stirring for at least 30 minutes, with 1.40 ml of sterile chloroform and with 108 mg of sterile paclitaxel (titre > 99%) in powder form.

The mixture is processed in a homogenizer (suitably sterilized) at high pressure (9000-40000 psi) until a nanoemulsion (MPS < 0.2 microns) is obtained, this being rapidly frozen to -80°C and lyophilized for 55 hours under sterile conditions, while raising the temperature to +30°C.

The powder obtained, containing 4.83 % (w/w) of paclitaxel and 4% (w/w) of water, is reconstituted with an aqueous 0.9% NaCl solution to a paclitaxel concentration of 2 mg/ml. The formulation obtained has an MPS of 0.175 microns, pH=5.6, and a stability > 24 hours.

Equivalent results are obtained by using phosphoric acid instead of hydrochloric acid.

It is important to remark that the use of sterile paclitaxel in powder form enables to achieve the important advantage that only one reactor is required for forming the liquid mixture containing HSA and paclitaxel with consequent reduction of costs and time necessary for completing the process.

### EXAMPLE 6

### Preparation of a formulation with citric acid and paclitaxel in powder form

An injectable aqueous 25% (w/v) HSA solution in accordance with FDA specifications is diluted to 3% (w/v) with sterile demineralized water, the pH being corrected to a value of 5.4 with citric acid which salifies some basic groups present in albumin.

50 ml of said solution, previously sterilized, are mixed under vigorous stirring for at least 40 minutes, with 1.23 ml of sterile chloroform and with 98 mg of sterile paclitaxel (titre > 99%) in powder form.

The mixture is processed in a homogenizer (suitably sterilized) at high pressure (9000-40000 psi) until a nanoemulsion (MPS < 0,2 microns) is obtained, this being rapidly frozen to -30°C and lyophilized for 57 hours under sterile conditions, while raising the temperature to +35°C.

The powder obtained, containing 4.80 % (w/w) of paclitaxel and 3.8% (w/w) of water, is reconstituted with an aqueous 0.9% NaCl solution to a paclitaxel concentration of 2 mg/ml. The formulation obtained has an MPS of 0.19 microns, pH=5.4, and a stability > 24 hours.

Equivalent results are obtained by using acetic acid instead of citric acid.

### EXAMPLE 7

### Preparation of a formulation with sterile citric acid and paclitaxel in powder form.

An injectable aqueous 25% (w/v) HSA solution in accordance with FDA specifications is diluted to 3% (w/v) with sterile demineralized water, the pH being corrected to a value of 5.5 with sterile citric acid which salifies some basic groups present in albumin.

37 ml of said solution are mixed under vigorous stirring for at least 40 minutes, with 0.91 ml of sterile chloroform and 71 mg of sterile paclitaxel (titre > 99%) in powder form, after which the mixture is cooled to 5-8°C.

The mixture is processed in a homogenizer (suitably sterilized) at high pressure (9000-40000 psi) until a nanoemulsion (MPS < 0.2 microns) is obtained, this being rapidly frozen to -80°C and lyophilized for 58 hours under sterile conditions, while raising the temperature to +30°C.

The powder obtained, containing 4.70 % (w/w) of paclitaxel and 4.5% (w/w) of water, is reconstituted with an aqueous 0.9% NaCl solution to a paclitaxel concentration of 2 mg/ml. The formulation obtained has an MPS of 0.185 microns, pH=5.5, and a stability > 24 hours.

### EXAMPLE 8

### Preparation of a formulation containing 9.36% of paclitaxel

An injectable aqueous 25% HSA solution in accordance with FDA specifications is diluted to 3% (w/v) with sterile demineralized water, the pH being corrected to 5.6 with 1M HCl which salifies some basic groups present in albumin. 60 ml of said solution, suitably sterilized, are mixed with 2.15 ml of a sterile solution of 110 mg/ml of paclitaxel in CHCl₃, after which the mixture is processed in a homogenizer (suitably sterilized) at high pressure (9000-40000 psi) until a nanoemulsion (MPS < 0.2 microns) is obtained, this being frozen to -50°C and lyophilized for 50 hours under sterile conditions, while raising the temperature to +30°C.

The powder obtained, containing 9.36% of paclitaxel and 3.9% (w/w) of water, is reconstituted with an aqueous 0.9% NaCl solution to a paclitaxel concentration of 2.5 mg/ml. The formulation obtained has an MPS of 0.175 microns, pH=5.6, and a stability > 24 hours.

### EXAMPLE 9

### Preparation of formulation at pH 5.5

An injectable aqueous 20% (w/v) HSA solution in accordance with FDA specifications (pH=6.9±0.5) is diluted to 3% (w/v) with sterile demineralized water, the pH being corrected to a value of 5.5 with citric acid which salifies some basic groups present in albumin.

110 ml of said solution are mixed with 4.10 ml of sterile CHCl₃ and with 639 mg of sterile paclitaxel (titre > 99%) in powder form, then the mixture is processed in a high pressure homogenizer (suitably sterilized) until a nanoemulsion (MPS about 0.2 microns) is obtained, this being filtered through a sterile filter (0.2 microns), evaporated under vacuum to remove the solvents, frozen and lyophilized under sterile conditions for 48 hours.

The powder obtained, containing 10.8 % (w/w) of paclitaxel, is reconstituted with an aqueous 0.9% NaCl solution to a paclitaxel concentration of 2 mg/ml. The formulation obtained has an MPS of 0.15 microns and a stability > 24 hours.

## Claims

1. An antitumor formulation consisting of a lyophilized powder of nanoparticles of paclitaxel and human serum albumin, in which the paclitaxel is present in a quantitity between 1% and 20% and the albumin between 60% and 98%, the percentages being by weight and the mean nanoparticle size being less than 0.2 microns, **characterized in that** said lyophilized powder contains between 1% and 20% by weight of biocompatible salts obtained by salification of at least one bicompatible acid or due to the presence of at least one biocompatible acid buffer substance, the acid or the buffer substance being present in a quantity such that the pH of a reconstituted aqueous injectable mixture of the powder is between 5.4 and 5.8 .

2. A formulation as claimed in claim 1, **characterized in that** said pH is between 5.5 and 5.7.

3. A formulation as claimed in claims 1 and 2 **characterized in that** said lyophilized powder contains up to 5% (w/w) of water.

4. A formulation according to claims 1 to 3, **characterized in that**, when reconstituted to form a physiological injectable mixture, it contains paclitaxel at a concentration between 0.1 and 3 mg/ml.

5. Injectable aqueous mixture of a formulation according to claims 1 to 4, **characterized in that** it contains paclitaxel at a concentration between 0.1 and 3 mg/ml.

6. Injectable aqueous mixture as claimed in claim 5, **characterized in that** paclitaxel is present at a concentration between 0.5 and 2.5 mg/ml.

7. Physiological injectable mixture obtainable from an antitumor formulation according to any of the preceding claims.
